Europäisches Patentamt

European Patent Office

Office européen des brevets

(19)

(11) **EP 1 226 810 B1**

(12) **EUROPEAN PATENT SPECIFICATION**

(45) Date of publication and mention
of the grant of the patent:
**31.08.2005 Bulletin 2005/35**

(51) Int Cl.⁷: **A61K 7/00**, A61K 47/36

(21) Application number: **01101601.1**

(22) Date of filing: **25.01.2001**

(54) **Use of a composition for entrapping oil and/or fat and the composition itself**

Zusammensetzung für den Einschluss von Öl und/oder Fett sowie ihre Verwendung zu diesem Zweck

Composition pour le piégeage d'huile et/ou de matière grasse et l'utilisation de la composition à cet effet

(84) Designated Contracting States:
**AT BE CH CY DE DK ES FI FR GB GR IE IT LI LU MC NL PT SE TR**

(43) Date of publication of application:
**31.07.2002 Bulletin 2002/31**

(73) Proprietor: **The Jordanian Pharmaceutical Manufacturing and Medical Equipment Co.Ltd. Jo-Naor 11710 (JO)**

(72) Inventors:
• **Badwan, Adnan Ali, Dr. Amman (JO)**
• **Amro, Basam I. Amman 11181 (JO)**
• **Nazzal, Hala G. Tarek Area Amman (JO)**

(74) Representative: **Goddar, Heinz J. FORRESTER & BOEHMERT Pettenkoferstrasse 20-22 80336 München (DE)**

(56) References cited:
**US-A- 5 637 291**

• **CHU C -H ET AL: "PH-SENSITIVE SWELLING OF A POLYELECTROLYTE COMPLEX GEL PREPARED FROM XANTHAN AND CHITOSAN" BIOSCIENCE BIOTECHNOLOGY BIOCHEMISTRY,JP,JAPAN SOC. FOR BIOSCIENCE, BIOTECHNOLOGY AND AGROCHEM. TOKYO, vol. 59, no. 4, 1 April 1995 (1995-04-01), pages 717-719, XP000511627 ISSN: 0916-8451**
• **GENNARO A.R.: 'Remington: The Science and Practice of Pharmacy', 1995, MACK PUBLISHING COMPANY, EASTON PENNSYLVANIA * page 289 - page 291 ***
• **FIEDLER H.P.: 'Lexikon der Hilfsstoffe für Pharmazie, Kosmetik, und angrenzende Gebiete', AULENDORF: ECV, AULENDORF XPBK: FIEDLE * page 130 - page 132 * * page 349 - page 351 * * page 1682 ***

## Description

The invention relates to the use of a composition for entrapping oil and/or fat and the composition itself.

The decomposition of chitin using alkali results in acetates and the weak basic, deacetylated and partly depolymerized crystallizable chitosan.

Chitosan has been reported widely in the literature to bind fat, see for example Hennen, W.J., 1996, *Chitosan*, WoodLand Publishing Inc. Many additives have been tried to enhance the ability of chitosan to bind fat such as ascorbic acid and hydroxy citric acid, see for example Lehoux, J.G. and Grondin, F. 1993, Some effects of Chitosan on liver function in the rat, *Endocrinology,* 132: 1078-1084. Other fibers have been tried similar to chitosan, such as cellulose, Guar gum, etc., see Anderson J.W. and Siesel, A.E., 1990, Hypocholesterolemic effects of oat products, in: Furda, I. and Brine, C.J. (editors), *New developments in dietary fiber,* Plenum press, New York, USA, 17-36.

Moreover, alginate was mixed with chitosan to produce a matrix for different purposes. Usually, this matrix was directed towards tablet formulation for drug release, see Fattah, E.A.E1., Grant, D.J.W., Gabr, K.E. and Meshali, M.M., 1998, Physical characteristics and release behavior of salbutamol sulfate beads prepared with different ionic polysaccharides, *Drug Development and Industrial Pharmacy*, 24(6):541-547; Miyazaki, T., Yomota, C. and Okada, S., 1990, Usage of chitosan as a pharmaceutical materiel effectiveness as an additional additive of sodium alginate, *Eisei Shikenjo Hokoku,* 108; 95-97 "abstract only"; Yomota, C., Miyazaki, T. and Okada, S., 1994, Sustained release effect of the direct compressed tablets based on chitosan and sodium alginate, *Yakugaka Zasshi*, 114(4); 257-263 "abstract only".

Finally, xanthan has been combined with chitosan for producing pH sensitive swelling gels, see Chia-Hong Chu et al. 1995, pH-Sensitive Swelling of a Polyelectrolyte Complex Gel Prepared from Xanthan and Chitosan, Biosci. Biotech. Biochem. 59(4), 717-719.

The ability of entrapping oil and/or fat by chitosan is not as satisfying as possible.

Therefore it is an object of the present invention to overcome the drawbacks of the prior art, especially to provide a composition comprising chitosan which can be used to enhance the ability of chitosan to bind fat.

One aspect of the invention is a composition for entrapping oil and/or fat comprising simulated gastric fluid as a solvent; chitosan, wherein chitosan is present in the solvent in a range from about 0,75 to about 2,00 % (w/v), preferably from about 1,5 to about 2,00 % (w/v); and sodium alginate. The pH of the composition is in a range from about 1 to about 6, preferably from about 3 to about 5,5.

Further a composition is an aspect of the invention, wherein sodium alginate is present in the composition in a range from about 0,05 to about 0,8 % (w/w), preferably from about 0,2 to about 0,6 % (w/w).

Still another aspect of the invention is a composition, wherein xanthan gum is additionally present in the composition, preferably in a range from about 0,4 to about 2,00 % (w/w), more preferably from about 1,0 to about 1,7 % (w/w).

Further, a composition is an aspect of the invention, wherein the composition further comprises additives.

The additives may comprise buffer, emulsifier and the like.

Still another aspect of the invention is a composition, wherein the composition further comprises oil and/or fat.

Finally, it is an aspect of the invention to use the composition according to the invention for entrapping oil and/or fat in an emulsion.

Surprisingly, it was found that a composition according to the present invention can enhance the viscosity of an emulsion comprising the composition and oil and/or fat and can prevent oil or fat from leaking from the emulsion.

Other objects, features, and advantages of the present invention will become apparent from the following detailed description and drawings, wherein

Figure 1    is a diagrammatic representation of the effect of pH of chitosan solution on emulsion viscosity and percent of leaked oil induced by centrifugation method;

Figure 2    is a diagrammatic representation of the effect of chitosan solution concentration on emulsion viscosity;

Figure 3    is a diagrammatic representation of the effect of sodium alginate on emulsion viscosity and percent of leaked oil; and

Figure 4    is a diagrammatic representation of the effect of xanthan gum on emulsion viscosity and percent of leaked oil.

A composition according to the present invention can be prepared as follows:

1. Preparation of Chitosan solution

A chitosan solution of a required concentration (in % (w/v)) was prepared by dissolving a suitable amount of chitosan in a suitable volume of simulated gastric fluid to obtain the required concentration. The chitosan solution was shaken overnight in a shaking water bath at 37° C. Then, the chitosan solution was filtered through gauze and was ready for use.

Chitosan was used in concentrations in the range from about 0,75 to about 2,00 % (w/v). In the vicinity of a concentration of about 2,00 % (w/v) the concentration of the chitosan solution is close to saturation and its pH is about 5,4.

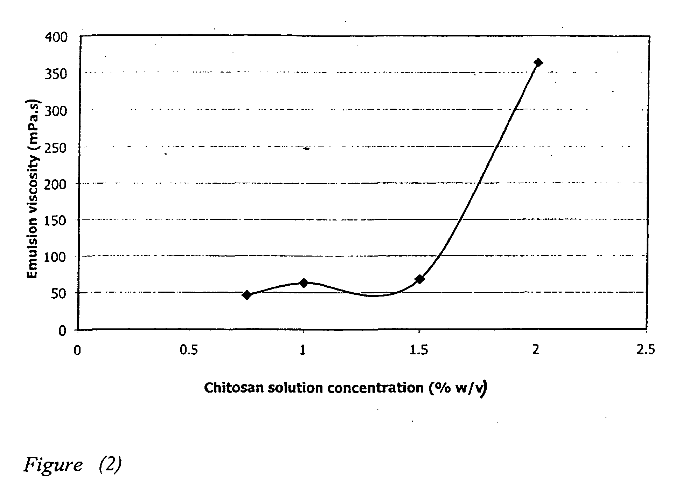

As can be seen in Figure 2, a concentration of greater than 1,5 % (w/v) of a chitosan aqueous solution results in a marked increase in the solution viscosity. A high solution viscosity seems to be responsable for an effectiv oil entrappment.

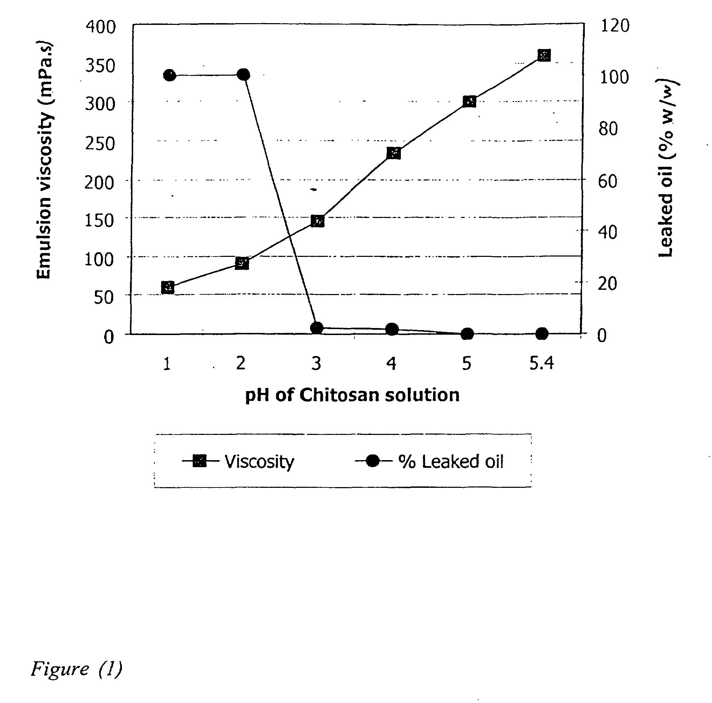

The pH of the chitosan solution corresponds to the concentration of chitosan in the simulated gastric fluid. Therefore, as can be seen in figure 1, with increasing pH also the emulsion viscosity increases with increasing pH.

2. Preparation of an emulsion of the chitosan solution and oil and characterization thereof

An emulsion of the required concentration (in % (w/w)) was prepared by mixing suitable weights of oil and 2,00 % (w/v) of chitosan solution to obtain the required concentration of oil in the emulsion. Emulsification was carried out by mixing the components at 2000 rpm for ten minutes at room temperature. The concentration of oil in the emulsion was calculated using the following formula:

$$\text{Concentration of oil in emulsion (in \% (w/w))} = \frac{\text{Weight of oil}}{\text{Weight of total emulsion}} \times 100$$

The prepared emulsion can be characterized as follows:

(A) Visual examination:

100 ml of the prepared emulsion was placed in an Erlenmeyer flask at 25°C for one hour. Any separation of oil layer or droplets from emulsion, emulsion fluidity and flocculates formation were inspected.

(B) Microscopic examination:

A sample of the emulsion was spread on a microscopic slide, covered by a slide cover and examined under a Nikon microscope at 40X magnification power. The size of the droplets, their crowdness and coalescence were inspected.

(C) Viscosity measurements

The viscosity of 50 ml of samples was measured by rotational viscosimeter (Haake, RV 12) at 25,0 + 0,1°C at a shearing rate of 500 rpm for 2 minutes. Every measurement was repeated three times.

(D) Analysis of leaked oil

Oil leakage was induced by two methods:

I. Addition of 10 % (w/v) aqueous solution of sodium cholate

This technique was applied only to stable emulsions, which showed no separation of any oil droplets. This is to ensure that the leakage of oil is due to the addition of sodium cholate and not due to instability of the prepared emulsion.

5 ml of 10 % (w/v) aqueous solution of sodium cholate was added to 25 g of emulsion, the mixture was then shaken for 15 minutes at 200 rpm at room temperature.

The leaked oil was filtered and extracted three times with chloroform (50 ml chloroform in each extraction step) and then titrated with 0,1M ethanolic solution of KOH, using 0,5 ml of phenolphthalein solution as indicator.

The endpoint represented the volume of 0,1M ethanolic solution of KOH consumed by free fatty acids found in the portion of oil leaked from the emulsion. The end point was used as indicative parameter to evaluate the entrapment ability of emulsion under testing.

II. Centrifugation of emulsion (accelerated stability studies)

50ml of emulsion was centrifuged by Hermele Z centrifuge at 4000 rpm for 10 minutes. This method was used for emulsions which showed separation of oil droplets at its surface. The leaked oil layer was decanted from emulsion and was then weighed. The percent of leaked oil was calculated relative to the total weight of oil used in emulsion preparation.

Table I gives a comparison between the two methods of analysis of leaked oil clearly indicating that the sodium cholate method is to be preferred.

Table I

| Measurement of olive oil leakage at pH 5,4 from an emulsion of equivalent quantities of 2%(w/v) chitosan solution and olive oil. | |
| --- | --- |
| Leakage inducing method | %(w/w) leaked oil |
| Centrifugation | 0.0 |
| Sodium cholate | 39.0 |

Figure 1 shows that with increasing pH of the chitosan solution the percentage of leaked oil (% w/w) in an emulsion comprising chitosan solution and oil, decreases dramatically at a pH of 3 by employing the centrifugation method described above. However, as shown in table I the use of sodium cholate method to induce and consequently determine leaked oil was much more effective in measuring leaked oil. Therefore, all experiments were carried out using the sodium cholate method described above.

3. Addition of sodium alginate to the emulsion of chitosan solution and oil

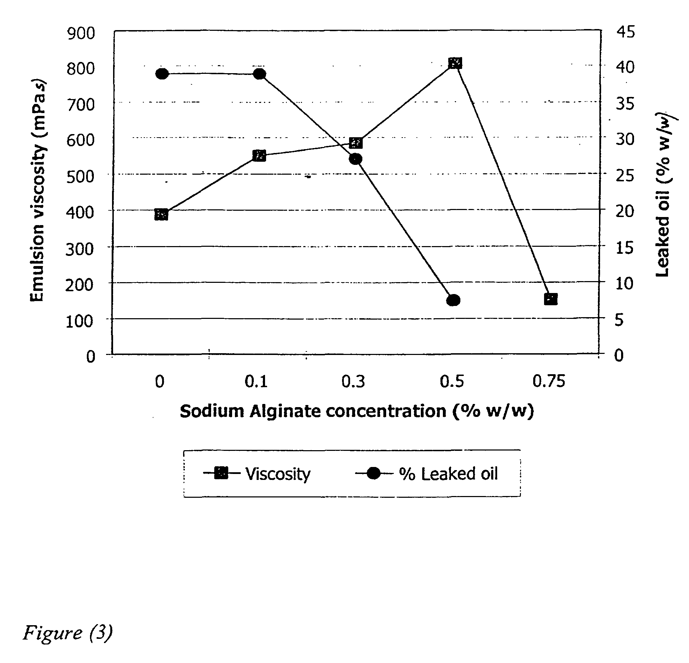

Figure 3 shows the effect of sodium alginate on emulsion viscosity and percentage of leaked oil. As can be seen, sodium alginate which is the hydrophilic polymer according to the present invention is very effective in improving chitosan oil entrapment. If 0,5% (w/w) sodium alginate are added to 2% (w/v) chitosan simulated gastric fluid and oil the viscosity of the resulting emulsion increased to 800mPa x s. This value for viscosity is more than twice as high as for an emulsion comprising chitosan solution and oil exclusively.

Figure 3 demonstrates that addition of sodium alginate with a concentration greater that 0,1% (w/w) to a chitosan solution improves markedly the ability of chitosan to trap oil. This is reflected by the increase in the viscosity of the resulting chitosan - alginate - oil emulsion. As well, the ability of the resulting emulsion to hold the mixed oil is significant. About 90% (w/w) of the added oil can be held using a chitosan solution in simulated gastric fluid with sodium alginate in a concentration of 0,5% (w/w) in the solution. The resulting emulsion preserved a stable cream like status over many hours.

4. Addition of xanthan gum to the emulsion of chitosan solution and oil (comparative example)

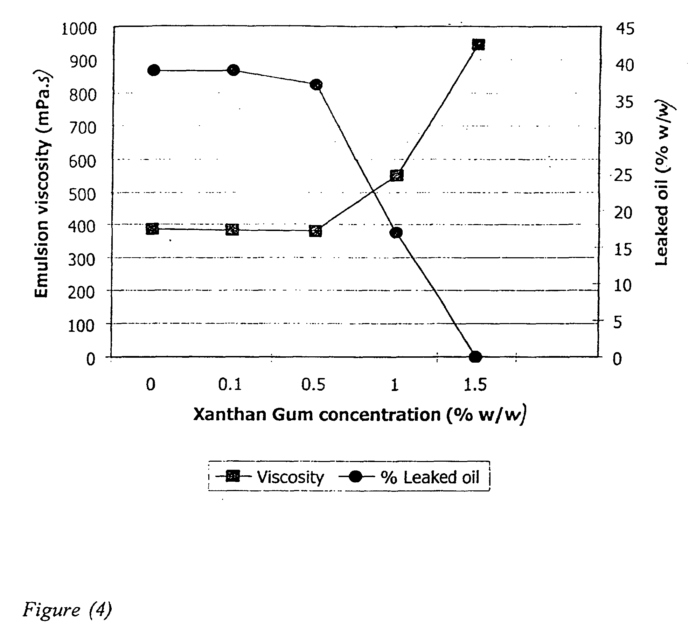

Figure 4 shows that adding 1,5% (w/w) xanthan gum to a 2% (w/v) chitosan solution in simulated gastric fluid and oil results in an increase of viscosity of the resulting emulsion to 950 mPa x s. The resulting stable cream like emulsion helds the added oil by 100% entrapment at such xanthan gum concentrations.

Already addition of 0,5% (w/w) xanthan gum to the chitosan solution decreases the percentage of leaked oil.

All experiments listed in figures 3 and 4 were conducted at a pH of 5,4.

Therefore, with the present invention the chitosan ability to oils and fats in vitro can be accessed.

The features disclosed in the foregoing description, in the claims and in the accompanying drawings may, both separately and in any combination thereof, be material for realizing the invention in diverse forms thereof.

**Claims**

1.   Composition for entrapping oil and/or fat comprising:

    -    simulated gastric fluid as a solvent, wherein the pH of the composition is in a range from about 1 to about 6;

- chitosan, wherein chitosan is present in the solvent in a range from about 0,75 to about 2,00 % (w/v), preferably from about 1,5 to about 2,00 % (w/v); and

- sodium alginate.

2. Composition according to claim 1, wherein sodium alginate is present in the composition in a range from about 0,05 to about 0,8 % (w/w), preferably from about 0,2 to about 0,6 % (w/w).

3. Composition according to claim 1 or 2, wherein the composition additionally contains xanthan gum.

4. Composition according to claim 3, wherein xanthan gum is present in the composition in a range from about 0,4 to about 2,00 % /w/w), preferably from about 1,0 to about 1,7 % (w/w).

5. Composition according to any of the preceding claims, wherein the pH of the composition is in a range from about 3 to about 5,5.

6. Composition according to any of the preceding claims, wherein the composition further comprises additives.

7. Composition according to claim 6, wherein the additives comprise buffer, emulsifier.

8. Composition according to any of the preceding claims, wherein the composition further comprises oil and/or fat.

9. Use of the composition according to any of the claims 1 to 8, for entrapping oil and/or fat in an emulsion.

**Patentansprüche**

1. Zusammensetzung für den Einschluß von Öl und/oder Fett, welche umfaßt:

- simulierte Magenflüssigkeit als ein Lösungsmittel, wobei der pH-Wert der Zusammensetzung in einem Bereich von etwa 1 bis etwa 6 ist;

- Chitosan, wobei Chitosan im Lösungsmittel in einem Bereich von etwa 0,75 bis etwa 2,00 % (w/v), bevorzugt von etwa 1,5 bis etwa 2,00 % (w/v) vorliegt; und

- Natriumalginat.

2. Zusammensetzung nach Anspruch 1, wobei Natriumalginat in der Zusammensetzung in einem Bereich von etwa 0,05 bis etwa 0,8 % (w/w), bevorzugt von etwa 0,2 bis etwa 0,6 % (w/w) vorliegt.

3. Zusammensetzung nach Anspruch 1 oder 2, wobei die Zusammensetzung zusätzlich Xanthan enthält.

4. Zusammensetzung nach Anspruch 3, wobei Xanthan in der Zusammensetzung in einem Bereich von etwa 0,4 bis etwa 2,00 % (w/w), bevorzugt von etwa 1,0 bis etwa 1,7 % (w/w) vorliegt.

5. Zusammensetzung nach einem der vorangehenden Ansprüche, wobei der pH-Wert der Zusammensetzung in einem Bereich von etwa 3 bis etwa 5,5 liegt.

6. Zusammensetzung nach einem der vorangehenden Ansprüche, wobei die Zusammensetzung ferner Additive umfaßt.

7. Zusammensetzung nach Anspruch 6, wobei die Additive Puffer und Emulgator umfassen.

8. Zusammensetzung nach einem der vorangehenden Ansprüche, wobei die Zusammensetzung ferner Öl und/oder Fett umfaßt.

9. Verwendung der Zusammensetzung nach einem der Ansprüche 1 bis 8 für den Einschluß von Öl und/oder Fett in einer Emulsion.

**Revendications**

1. Composition pour piéger l'huile et/ou les matières grasses comprenant :

   - un liquide gastrique simulé en tant que solvant, dans laquelle le pH de la composition est dans la plage comprise entre environ 1 et environ 6 ;
   - du chitosane, dans laquelle le chitosane est présent dans le solvant dans une plage comprise entre environ 0,75 et environ 2,00 % (p/p), de préférence entre environ 1,5 et environ 2,00 % (p/p) ; et
   - de l'alginate de sodium.

2. Composition selon la revendication 1, dans laquelle l'alginate de sodium est présent dans la composition dans une plage comprise entre environ 0,05 et environ 0,8 % (p/p), de préférence de préférence entre environ 0,2 et environ 0,6 % (p/p).

3. Composition selon la revendication 1 ou 2, dans laquelle la composition contient de plus de la gomme xanthique.

4. Composition selon la revendication 3, dans laquelle la gomme xanthique est présente dans la composition dans une plage comprise entre environ 0,4 et environ 2,00 % (p/p), de préférence entre environ 1,0 et environ 1,7 % (p/p).

5. Composition selon l'une quelconque des revendications précédentes, dans laquelle le pH de la composition est dans une plage comprise entre environ 3 et environ 5,5.

6. Composition selon l'une quelconque des revendications précédentes, dans laquelle la composition comprend en outre des additifs.

7. Composition selon la revendication 6, dans laquelle les additifs comprennent un tampon, un émulsifiant.

8. Composition selon l'une quelconque des revendications précédentes, dans laquelle la composition comprend en outre de l'huile et/ou des matières grasses.

9. Utilisation de la composition selon l'une quelconque des revendications 1 à 8, pour piéger l'huile et/ou les matières grasses dans une émulsion.

*Figure (1)*

*Figure (2)*

*Figure (3)*

Figure (4)